# EUROPEAN PATENT APPLICATION

(11) **EP 4 714 403 A1**
(43) Date of publication of application: **25.03.2026**
(21) Application number: 24201548.5
(22) Date of filing: 20.09.2024
(51) Int. Cl.: A61F 5/03, A61F 13/14

(54) **KINESIOLOGY TAPE FOR PROVIDING ABDOMINAL SUPPORT DURING PREGNANCY AND KINESIOLOGY TAPE SET**

(71) Applicant: Nanjing 3H Medical Products Co., Ltd., Nanjing (CN)
(72) Inventor: ZHU, Zhiyang, 211000 Nanjing (CN); FANG, Zhongyu, 211000 Nanjing (CN); ZHANG, Yunyi, 211000 Nanjing (CN)
(74) Representative: Seemann & Partner Patentanwälte mbB

(57) **Abstract**

The invention relates to a kinesiology tape (2) for providing abdominal support during pregnancy as well as a kinesiology tape set (1). The tape (2) is a flat fabric tape with an adhesive (33) on a lower side. The tape (2) is pre-cut into a sickle-shape with a concave side (10) and a convex side (20).

## Description

The invention relates to a kinesiology tape for providing abdominal support during pregnancy. The invention further relates to a kinesiology tape set.

During pregnancy, the expanding abdomen often leads to discomfort and pain in the lower back and abdominal regions. To alleviate these complaints, pregnant women often use maternity belts. However, maternity belts are often bulky, uncomfortable and may cause skin irritation due to prolonged contact with the skin. Thus, pregnant women often have to choose whether to endure the discomfort and pain or experience the discomfort of wearing a maternity belt for prolonged periods of time.

It is the object of the present invention to provide a way to alleviate the pain and discomfort in the abdominal region of pregnant women without causing skin irritations.

This object is solved by a kinesiology tape for providing abdominal support during pregnancy, whereas the tape is a flat fabric tape with an adhesive on a lower side, wherein the tape is pre-cut into a sickle shape with a concave side and a convex side.

Due to the sickle shape of the tape, it may be fitted easily to the abdomen in order to provide support. In this way, the tape helps to alleviate the pain and discomfort during pregnancy. The sickle shape form of the tape is important, as a straight tape would not be able to support the abdomen nearly as much. By being pre-cut into the sickle shape, the tape is easy to apply and to position correctly. The kinesiology tape may be worn during daily activities, providing continuous support and pain relief. Compared to a maternity belt, it is light-weighed, unobtrusive and can be easily worn under clothing without being visible.

The kinesiology tape according to the invention may be called mom-mytape or pregnancy support tape.

In particular, the tape is made from a soft and/or breathable fabric. The use of a soft breathable fabric lowers the discomfort even further.

Preferably, the concave side follows a circular arc with a diameter between 450 mm to 700 mm, in particular between 550 mm and 620 mm, further in particular between 560 mm and 610 mm, further in particular between 570 mm and 600 mm, and further in particular between 579 mm and 592 mm. In other words, the concave side of the tape conforms at least in section to a circular arc. The diameter of the circular arc is chosen so as to best support the abdomen. In order to best support the abdomen during different stages of pregnancy or the abdomen of different women, the tape may be pre-cut in different shapes and/or sizes. For example, a first embodiment of the tape may have a concave side that follows a circular arc with a diameter of 580 mm, while a second embodiment may have a concave side that follows a circular arc with a diameter of 591.5 mm.

Preferably, the tape comprises a middle line and two ends, which are a first end and a second end, wherein the middle line divides the tape into a first side comprising the first end and a second side comprising the second end, wherein in particular the first side and the second side are symmetrical. The middle line may just be an imaginary line or may comprise perforations. In both cases, the middle line divides the tape into the first side and the second side. By being symmetric to the middle line, the first side and the second side may be applied easily to the abdomen and provide a balanced support.

Preferably, a width of the tape is larger at the middle line than at the ends, wherein in particular the width at the middle line is between 50 mm and 150 mm, in particular between 60 mm and 120 mm, further in particular between 70 mm and 110 mm, and further in particular between 74 mm and 101 mm. In other words, the tape widens towards the middle. This shape of the tape offers further increased support for the expanding abdomen during pregnancy. The width at the middle line may be chosen to fit a particular abdomen. For example, in the first embodiment the width may be 100 mm, whereas in the second embodiment the width may be 75 mm.

Preferably, a diameter of a circular arc following the convex side increases from the ends towards the middle line, wherein in particular the diameter of the circular arc following the convex side increases from at most 60 mm at the ends to at least 600 mm at the middle line. In other words, the curvature of the convex side decreases, in particular monotonically, from the ends towards the middle line. This shape of the convex side best conforms to the abdomen of a pregnant woman and offer increased support.

Preferably, the ends of the tape are rounded ends, wherein in particular the rounded ends have the form of circle segments with a diameter of 20 mm to 80 mm, in particular between 30 mm and 70 mm, further in particular between 40 mm and 60 mm, and further in particular between 46 mm and 53 mm. The rounded ends in the form of circle segments are easy to apply on the abdomen and offer increased support.

Preferably, the adhesive is covered by a removable protective film, wherein the protective film is segmented into multiple sections, which are divided by perforation lines, wherein in particular the protective film is segmented into at least five sections. The removable protective film allows for an easy application of the tape. In particular, the removable protective film is an inelastic, tearable strip, for example an inelastic, tearable paper strip.

By segmenting the protective film into multiple sections, the protective film is easier to remove. This is especially beneficial, as the sickle shape of the tape would otherwise make the removal of the protective film harder. Five sections of the protective film are especially advantageous, as they make the application of the tape to the abdomen very easy.

In particular, the adhesive of the tape comprises anti-skin-irritating ingredients, in particular an acrylic adhesive. In particular, the adhesive comprises at least 60 %, in particular 75 - 90%, acrylic copolymer and at least 5 %, in particular 10 - 25 %, silicone.

Preferably, the tape comprises a polyether-polyurea copolymer fabric. In particular, the polyether-polyurea copolymer fabric is processed to form the material known as Spandex. The inclusion of this fabric offers some elasticity to the tape.

According to an embodiment, a weight per length of the polyether-polyurea copolymer fabric is less than 60 denier, in particular less than 50 denier, further in particular between 45 denier and 35 denier, wherein in particular polyether-polyurea copolymer fabric makes up 2.5 % to 3.5 % of the fabric of the tape. Denier is a unit of measurement for the linear mass density of fibers. It indicates the mass of the fiber per 9.000 meters of the fiber. The tape comprises a polyether-polyurea copolymer fabric with a comparatively low linear mass density of, for example, 40 denier. In particular, the weight per length of the polyether-polyurea copolymer fabric relates to the processed polyether-polyurea copolymer fabric, in particular Spandex. A low linear mass density increases the support to the abdomen. For the same reason, the polyether-polyurea copolymer fabric may make up, for example, 3 % of the fabric of the tape, as a higher value would decrease the support.

Preferably, the fabric of tape is stretchable to at most 145 % of its resting length before it rips. In other words, the kinesiology tape has a comparatively low elasticity. Other medical tapes often can be stretched to 200 % or more of their resting length. However, a low elasticity leads to a stronger support of the abdomen and is thus advantageous.

Preferably, a weight per unit area of the tape is between 120 g/m² and 190 g/m², in particular between 130 g/m² and 180 g/m², further in particular between 140 g/m² and 170 g/m², and further in particular between 150 g/m² and 160 g/m². For example, the weight per unit area is 155 g/m². This weight per unit area is slightly lower than the weight per unit area of usual medical tapes. This increases the comfort and offers increased support.

Preferably, the tape is a woven tape comprising multiple threads, wherein the threads comprise warps, which are longitudinally arranged side-by-side, and wefts, which are woven into the warps at a 90° angle to the warps, wherein a weight per length of the warps is bigger than a weight per length of the wefts. By using warps with a bigger weight per length than the wefts, the suitability of the tape as a kinesiology tape is increased. In particular, a density of the fabric of the tape is lower than 61*32. In particular the density of the fabric is 57*32. The numbers 61*32 and 57*32 refer to the number of warps and wefts per square foot or per square inch.

Preferably, a total length of the tape is at least 300 mm, in particular at least 350 mm. Such a length is advantageous in order to support the front of the abdomen from the sides. For example, the length is 400 mm in the first embodiment and 350 mm in the second embodiment. The length is measured from the first end to the second end in a straight line. The straight line is in particular octagonal to the middle line.

The object is further solved by kinesiology tape set for providing abdominal support during pregnancy, comprising a Kinesiology tape according to any of the previous embodiments, wherein the tape set further comprises two rectangular shaped tapes, wherein the rectangular shaped tapes are configured and formed to be applied on the lower dorsal or on the central ventral area.

The kinesiology tape set embodies the same advantages and characteristics as the kinesiology tape described above.

In particular, the rectangular shaped tapes have rounded ends. This makes the application and removal of the rectangular shaped tapes easier.

The tape set offers two primary configurations for the use during pregnancy. According to a full abdominal support configuration, both the sickle-shaped tape and the rectangular-shaped tapes are applied to the abdomen. The sickle-shaped tape is positioned on the lower abdomen or under the abdomen to provide lift and reduce strain on the abdominal muscles. The rectangular-shaped tapes are placed above the sickle-shaped tape on the abdomen, offering additional stability and support to the entire area.

In the other primary configuration, known as the abdominal and back support configuration, the sickle-shaped tape is applied to the lower abdomen or under the abdomen, just like in the previously described configuration. In this position, it provides targeted support to the abdominal area. However, the rectangular-shaped tapes are applied to the lower back area instead of the abdomen. This placement helps to alleviate lower back pain by providing support and stability to the back muscles, reducing the strain caused by the weight of the growing abdomen.

Further characteristics of the invention will become apparent from the description of the embodiments according to the invention together with the claims and the included drawings. Embodiments according to the invention can fulfil individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic simplified representation of a pregnant woman using a kinesiology tape set in the full abdominal support configuration in order to support the abdomen,
- Fig. 2: a schematic simplified representation of the pregnant woman using the tape set in an abdominal and back support configuration,
- Fig. 3: a schematic simplified representation of the front side of the sickle-shaped kinesiology tape,
- Fig. 4: a schematic simplified representation of the lower side of the sickle-shaped kinesiology tape, and
- Fig. 5: a schematic simplified representation of the sickle-shaped kinesiology tape with a number of auxiliary lines in order to describe the form and shape of the tape.

In the drawings, the same or similar types of elements or respectively corresponding parts are provided with the same reference numbers in order to prevent the item from needing to be reintroduced.

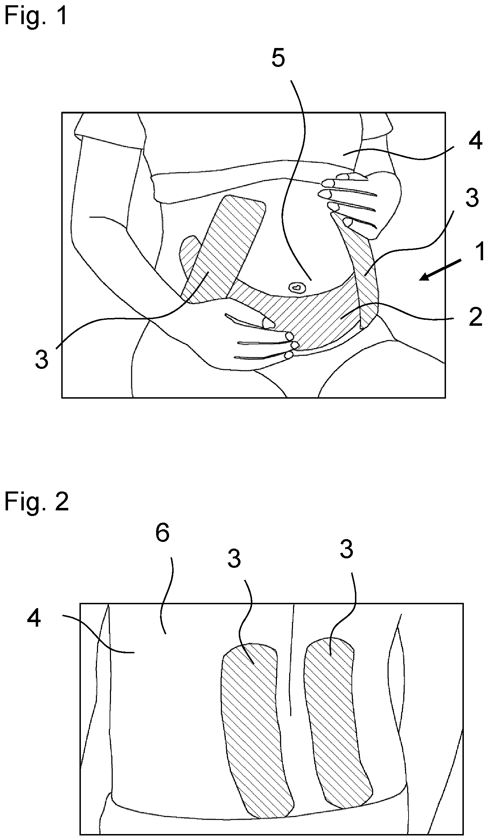

Fig. 1 shows a schematic simplified representation of a pregnant woman 4 using a kinesiology tape set 1 in order to support her abdomen 5 during pregnancy. The tape set 1 comprises a sickle-shaped kinesiology tape 2 as well as two rectangular-shaped tapes 3. In the full abdominal support configuration as shown in Fig. 1, the sickle-shaped kinesiology tape 2 is positioned on the lower abdomen or under the abdomen to provide lift and reduce strain on the abdominal muscles. The rectangular-shaped tapes 3 are placed across the sickle-shaped kinesiology tape 2 and offer additional stability and support.

Fig. 2 shows a schematic simplified representation of a different configuration called the abdominal and back support configuration. In this configuration, the sickle-shaped kinesiology tape 2 is placed on the lower part of the abdomen 5 or below the abdomen 5 as shown in Fig. 1. However, the rectangular-shaped tapes 3 are placed on the back 6 instead. More specifically, they are placed on the lower back in order to alleviate back pain and providing support and stability to the back muscles.

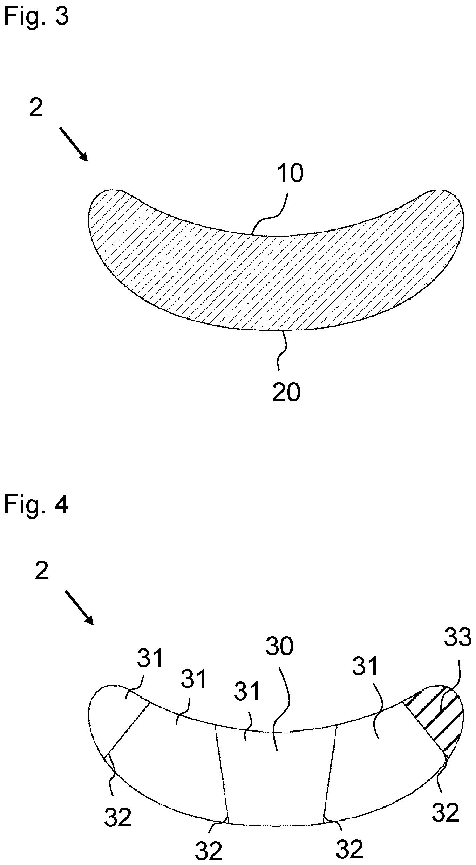

Fig. 3 shows a schematic simplified representation of the front side of the sickle-shaped kinesiology tape 2. As shown in Fig. 3, the kinesiology tape 2 has a concave side 10 and a convex side 20 as well as rounded ends. In addition, the width of the kinesiology tape 2 increased from the ends towards the middle. This increases the support offered by the kinesiology tape 2 greatly.

Fig. 4 shows the lower side or back side of the kinesiology tape 2 shown in Fig. 3. The lower side comprises an adhesive 33, which is covered by multiple sections 31 of a removable protective film 30. In Fig. 4, the right most one of the sections 31 is removed, so that the adhesive 33 is directly visible. The different sections 31 are separated by perforation lines 32. When applying the kinesiology tape 2 to the abdomen 5, the protective film 30 is removed so that the adhesive 33 comes into contact with the abdomen 5. The adhesive 33 is a medical grade acrylic adhesive developed to minimize the risk of skin irritation.

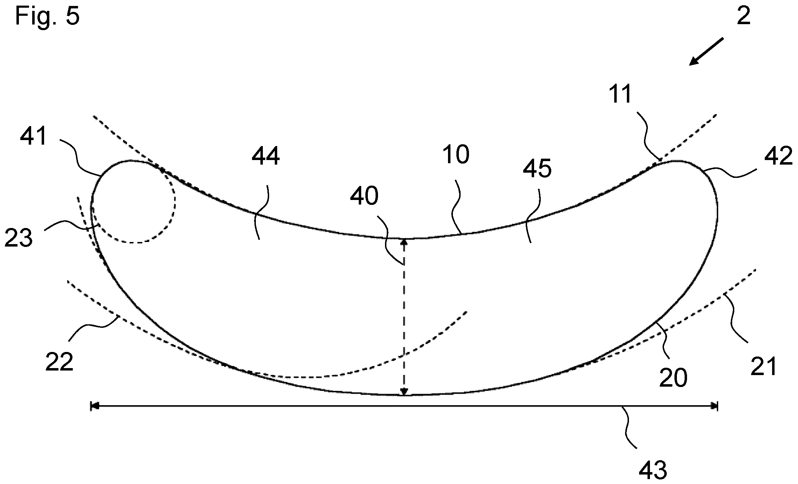

Fig. 5 shows another schematic simplified representation of the sickle-shaped kinesiology tape 2. A number of guiding lines are shown that highlight the shape of the kinesiology tape 2. As shown in Fig. 5, the kinesiology tape 2 extends from a first end 41 to a second end 42. The length 43, measured from the first end 41 to the second end 42 in a direct line, is, for example, 400 mm in a first embodiment and 350 mm in a second embodiment. A middle line 40 directly in between the ends 41, 42 divides the kinesiology tape 2 into a first side 44 and a second side 45. These sides 44, 45 are symmetrical to one another. The width of the kinesiology tape 2 at the middle line 40 may be 100 mm in the first embodiment and 75 mm in the second embodiment.

The concave side 10 follows largely a circular arc 11. The radius of this circular arc is, for example, 580 mm in the first embodiment and 591.5 mm in the second embodiment. The curvature of the convex side 20 decreases from the ends 41, 42 towards the middle line 40. At the ends 41, 42, it follows a circular arc 23 with a diameter of, for example, 52.5 mm in the first embodiment and 46.7 mm in the second embodiment. At a point in between the ends 41, 42 and the middle line 40, it follows another circular arc 22 with a diameter of 295.5 mm in the first embodiment and 226 mm in the second embodiment. At the middle line 40, it follows a third circular arc 21 with a diameter of, for example, 707 mm in the first embodiment and 726 mm in the second embodiment. These shapes and sizes are advantageous as they offer very good support to the abdomen 5 of the pregnant woman 20.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: tape set
- 2: tape
- 3: rectangular shaped tape
- 4: pregnant woman
- 5: abdomen
- 6: back
- 10: concave side
- 11: circular arc
- 20: convex side
- 21: circular arc
- 22: circular arc
- 23: circular arc
- 30: protective film
- 31: section
- 32: perforation line
- 33: adhesive
- 40: middle line
- 41: first end
- 42: second end
- 43: length
- 44: first side
- 45: second side

## Claims

1. Kinesiology tape (2) for providing abdominal support during pregnancy, whereas the tape (2) is a flat fabric tape with an adhesive (33) on a lower side, wherein the tape (2) is pre-cut into a sickle shape with a concave side (10) and a convex side (20).

2. Kinesiology tape (2) according to claim 1, **characterized in that** the concave side (10) follows a circular arc (11) with a diameter between 450 mm to 700 mm, in particular between 550 mm and 620 mm, further in particular between 560 mm and 610 mm, further in particular between 570 mm and 600 mm, and further in particular between 579 mm and 592 mm.

3. Kinesiology tape (2) according to claim 1 or 2, **characterized in that** the tape (2) comprises a middle line (40) and two ends, which are a first end (41) end a second end (42), wherein the middle line (40) divides the tape (2) into a first side (44) comprising the first end (41) and a second side (45) comprising the second end (42), wherein in particular the first side (44) and the second side (45) are symmetrical.

4. Kinesiology tape (2) according to claim 3, **characterized in that** a width of the tape (2) is larger at the middle line (40) than at the ends, wherein in particular the width at the middle line (40) is between 50 mm and 150 mm, in particular between 60 mm and 120 mm, further in particular between 70 mm and 110 mm, and further in particular between 74 mm and 101 mm.

5. Kinesiology tape (2) according to claim 3 or 4, **characterized in that** a diameter of a circular arc (21, 22, 23) following the convex side (20) increases from the ends towards the middle line (40), wherein in particular the diameter of the circular arc (21, 22, 23) following the convex side (20) increases from at most 60 mm at the ends to at least 600 mm at the middle line (40).

6. Kinesiology tape (2) according to any one of claims 3 to 5, **characterized in that** the ends of the tape (2) are rounded ends, wherein in particular the rounded ends have the form of circle segments with a diameter of 20 mm to 80 mm, in particular between 30 mm and 70 mm, further in particular between 40 mm and 60 mm, and further in particular between 46 mm and 53 mm.

7. Kinesiology tape (2) according to any one of claims 3 to 6, **characterized in that** the adhesive (33) is covered by a removable protective film (30), wherein the protective film (30) is segmented into multiple sections (31), which are divided by perforation lines(32), wherein in particular the protective film (30) is segmented into at least five sections (31).

8. Kinesiology tape (2) according to any one of claims 1 to 7, **characterized in that** the tape (2) comprises a polyether-polyurea copolymer fabric.

9. Kinesiology tape (2) according to claim 8, wherein a weight per length of the polyether-polyurea copolymer fabric is less than 60 denier, in particular less than 50 denier, further in particular between 45 denier and 35 denier, wherein in particular polyether-polyurea copolymer fabric makes up 2.5 % to 3.5 % of the fabric of the tape (2).

10. Kinesiology tape (2) according to any one of claims 1 to 9, **characterized in that** the fabric of tape (2) is stretchable to at most 145 % of its resting length before it rips.

11. Kinesiology tape (2) according to any one of claims 1 to 10, **characterized in that** a weight per unit area of the tape (2) is between 120 g/m² and 190 g/m², in particular between 130 g/m² and 180 g/m², further in particular between 140 g/m² and 170 g/m², and further in particular between 150 g/m² and 160 g/m².

12. Kinesiology tape (2) according to any one of claims 1 to 11, **characterized in that** the tape (2) is a woven tape comprising multiple threads, wherein the threads comprise warps, which are longitudinally arranged side-by-side, and wefts, which are woven into the warps at a 90° angle to the warps, wherein a weight per length of the warps is bigger than a weight per length of the wefts.

13. Kinesiology tape (2) according to any one of claims 1 to 12, **characterized in that** a total length of the tape (2) is at least 300 mm, in particular at least 350 mm.

14. Kinesiology tape set (1) for providing abdominal support during pregnancy, comprising a Kinesiology tape (2) according to any one of claims 1 to 13, wherein the tape set (1) further comprises two rectangular shaped tapes (3), wherein the rectangular shaped tapes (3) are configured and formed to be applied on the lower dorsal or on the central ventral area.
